# EUROPÄISCHE PATENTSCHRIFT

(11) **EP 3 301 680 B1**
(45) Veröffentlichungstag und Bekanntmachung des Hinweises auf die Patenterteilung: **18.12.2019**
(21) Anmeldenummer: 17192800.5
(22) Anmeldetag: 25.09.2017
(51) Int. Cl.: G16H 20/30, A61F 5/01

(54) **SYSTEM ZUR UNTERSTÜTZUNG DER PLANTARFLEXION IM OBEREN SPRUNGGELENK**
SYSTEM FOR ASSISTING PLANTAR FLEXION IN THE UPPER ANKLE JOINT
SYSTÈME DE SUPPORT DE LA FLEXION PLANTAIRE DANS LE JARRET SUPÉRIEUR

(30) Priorität: 30.09.2016 DE 102016219113
(43) Veröffentlichungstag der Anmeldung: 04.04.2018
(73) Patentinhaber: adViva GmbH, 69123 Heidelberg (DE)
(72) Erfinder: Biber, Gerhard, 69124 Eppelheim (DE)
(74) Vertreter: Kudla, Karsten

(56) Entgegenhaltungen:
- WO-A2-2015/088863

## Beschreibung

Die Erfindung geht aus von einem System zur Unterstützung der Plantarflexion im oberen Sprunggelenk, umfassend eine Unterschenkelorthese mit einem Fußteil mit einer Laufsohle sowie eine Manschette zur Befestigung der Unterschenkelorthese im Bereich des Schienbeinkopfs. Die Erfindung betrifft weiterhin ein Verfahren zur Unterstützung eines Patienten beim Gehen mit einem System zur Unterstützung der Plantarflexion im oberen Sprunggelenk.

Eine Unterschenkelorthese findet üblicherweise Einsatz, wenn der Bewegungsapparat insbesondere im Bereich des Fußgelenks nur eine eingeschränkte Funktion aufweist. Hierzu können organische Störungen oder Schädigungen der Muskulatur und Gelenke ursächlich sein oder auch Schädigungen im Gehirn oder Rückenmark in Bereichen, in denen die Bewegung gesteuert wird.

Für den Einsatz einer Unterschenkelorthese können viele Indikationen ursächlich sein. Mögliche Ursachen sind zum Beispiel eine Zerebralparese, Multiple Sklerose, Muskeldystrophie, neurale Muskelatrophie, Polyneuropathie, ein Schädel-Hirn-Trauma oder eine Apoplexie. Daneben werden Unterschenkelorthesen auch bei Verletzungen des Sehnen- und Bandapparates im Bereich des Sprunggelenks eingesetzt.

Einfache Unterschenkelorthesen bieten einen Halt des Fußes und eine mechanische Bewegungseinschränkung, damit der Fuß beim Gehen nicht nach unten hängt. Diese Orthesen können jedoch die Bewegung nicht unterstützen und führen insbesondere nicht zu einer Verbesserung des Gangbildes. Inzwischen werden jedoch vielfach Unterschenkelorthesen eingesetzt, die eine aktive Unterstützung beim Gehen bieten. Derartige Unterschenkelorthesen haben in der Regel eine Feder, die bei einem bestimmten Winkel des Fußes beim Abrollen auslöst und auf diese Weise die Bewegung unterstützt. Hierzu kann zum Beispiel wie aus DE-A 10 2011 082 700 bekannt eine Unterschenkelorthese eingesetzt werden, bei der zwischen Fußauflage und Bodenauflage ein elastischer Energiespeicher angeordnet ist. Eine in Abhängigkeit der Bewegung des Patienten veränderbare Federrate bei einer Unterschenkelorthese mit einer Stützfeder zwischen einer Manschette, mit der die Unterschenkelorthese am Fuß befestigt ist, und einer zweiten Manschette zur Befestigung am Unterschenkel ist aus DE-A 10 1004 030 570 bekannt. Die Veränderung der Federrate erfolgt dabei mit Hilfe einer elektronischen Rückstelleinrichtung.

WO 2015/088863 A2 offenbart einen flexiblen, unterstützenden Anzug mit Sensoren für einen Patienten zur Identifizierung von vordefinierten Gangmustern zum Zwecke der Gangrehabilitation. Ein Betätigungsprofil eines Aktuators wird kontinuierlich angepasst. Damit wird die Plantar- und/ oder Dorsalflexion des Fußes des Patienten unterstützt.

Nachteil aller aus dem Stand der Technik bekannten Systeme zur Unterstützung der Plantarflexion ist, dass für eine dauerhafte Verbesserung des Gangbildes eine intensive und langwierige physiotherapeutisch unterstützte, stationäre oder ambulante Übungsphase erforderlich ist.

Aufgabe der vorliegenden Erfindung war es daher, ein System zur Unterstützung der Plantarflexion im oberen Sprunggelenk mit eine Unterschenkelorthese bereitzustellen, das auch ein Üben zur Verbesserung des Gangbildes außerhalb einer stationären oder ambulanten Behandlung mit Korrektur des Gangbildes durch einen Physiotherapeuten erlaubt.

Diese Aufgabe wird gelöst durch ein System zur Unterstützung der Plantarflexion im oberen Sprunggelenk, umfassend eine Unterschenkelorthese mit einem Fußteil mit einer Laufsohle sowie eine Manschette zur Befestigung der Unterschenkelorthese im Bereich des Schienbeinkopfs, wobei das Fußteil und die Manschette mit einer den Unterschenkel an der Beinhinterseite umschließenden Feder verbunden sind, wobei an der Unterschenkelorthese Sensoren zur Erfassung eines Bewegungsmusters positioniert sind und das System eine Auswerteeinheit umfasst, die derart eingerichtet ist, dass von den Sensoren erfasste Daten zur Erfassung des Bewegungsmusters mit Solldaten verglichen werden und bei Abweichung eine Information an den Träger der Unterschenkelorthese gegeben wird, dadurch gekennzeichnet, dass die Feder (7) im Bereich des äußeren Knöchels mit dem Fußteil verbunden ist, den Unterschenkel an der Beinhinterseite umläuft und auf der Beininnenseite mit der den Unterschenkel im Bereich des Schienbeinkopfs umschließenden Manschette verbunden ist.

Der Einsatz von Sensoren erlaubt es, zusätzlich zur stützenden Funktion der Unterschenkelorthese den Bewegungsablauf während des Gehens oder Laufens zu erfassen. Durch den Vergleich der während der Bewegung mit den Sensoren erfassten Daten mit Solldaten, die einem bestmöglichen Bewegungsmuster entsprechen, ist es möglich, automatisiert eine Rückmeldung an den die Unterschenkelorthese tragenden Patienten zu generieren, mit der dem Patienten mitgeteilt wird, ob die aktuell ausgeführte Bewegung dem gewünschten Bewegungsmuster entspricht. Durch die unmittelbare Rückmeldung besteht die Möglichkeit, bereits beim nächsten Schritt eine Korrektur im Bewegungsablauf vorzunehmen. Auf diese Weise kann das Gehen mit einem optimierten Bewegungsmuster auch ohne Anwesenheit eines Therapeuten und dessen Rückmeldung geübt werden. Dies hat den Vorteil, dass das bestmöglich zu erreichende Bewegungsmuster nicht nur während einzelner Therapiestunden geübt werden kann sondern zu jeder beliebigen Zeit auch außerhalb der Therapiestunden.

Um ein möglichst umfassendes Bewegungsmuster zu erfassen, können unterschiedliche Arten von Sensoren eingesetzt werden. Auch ist es vorteilhaft, die Sensoren an verschiedenen Positionen der Unterschenkelorthese anzubringen. Als Sensoren zur Erfassung von Daten zur Bestimmung des Bewegungsmusters eignen sich zum Beispiel Drucksensoren, Biegesensoren, Dehnungssensoren, Bewegungssensoren und Elektroden zur Messung der elektrischen Muskelaktivität. Für ein möglichst genaues Bewegungsmuster ist es weiterhin vorteilhaft, wenn mehrere unterschiedliche Sensortypen eingesetzt werden. Hierbei ist es weiterhin von der Position an der Unterschenkelorthese abhängig, welche Arten von Sensoren sinnvollerweise verwendet werden.

So ist es zum Beispiel vorteilhaft, Drucksensoren im Bereich der Laufsohle anzubringen. Mit den Drucksensoren kann dann der Anpressdruck des Fußes auf dem Boden und damit auch eine Änderung des Druckverlaufs während des Abrollens des Fußes erfasst werden. Zusätzlich zu den Drucksensoren im Bereich der Laufsohle ist es auch möglich, weitere Drucksensoren an anderen Stellen der Unterschenkelorthese anzubringen, wobei diese dann insbesondere genutzt werden können, um einen sich ändernden Druck des Beins auf die Unterschenkelorthese zu erfassen. Entsprechende Sensoren dienen dann jedoch primär auch der Erfassung Passgenauigkeit und des exakten Anlegens der Unterschenkelorthese.

Um neben dem Druck beim Aufsetzen und Abrollen des Fußes auch weitere Bewegungsdaten zu erhalten, ist es vorteilhaft, zum Beispiel Bewegungssensoren, insbesondere 3D-Bewegungssensoren oder auch Beschleunigungssensoren an der spiralförmig den Unterschenkel umschließenden Feder und gegebenenfalls auch an der Manschette zur Befestigung der Unterschenkelorthese im Bereich des Schienbeinkopfes anzubringen. Zusätzlich oder alternativ können insbesondere im Bereich der Feder auch Dehnungssensoren angebracht werden, mit denen die Verformung der Feder während des Gehens oder Laufens erfasst wird.

Weiterhin kann es vorteilhaft sein, zusätzlich Elektroden zur Messung der elektrischen Muskelaktivität zu nutzen, wobei diese zum Beispiel an der Manschette zur Befestigung der Unterschenkelorthese im Bereich des Schienbeinkopfes oder an der Feder angebracht sein können. Durch die Erfassung der Muskelaktivität lässt sich nachvollziehen, welche Muskeln beim Gehen genutzt werden und in welcher Reihenfolge und Intensität der Muskeleinsatz erfolgt. Hierdurch kann auch auf das daraus resultierende Gangbild rückgeschlossen werden und bei einer falschen Reihenfolge im Muskeleinsatz oder einer falschen Intensität der Nutzung einzelner Muskeln eine Korrektur des Gangbildes durch entsprechende Rückmeldung an den Patienten erfolgen.

Zur Erfassung der Bewegung im oberen Sprunggelenk, beim Gehen und Laufen, oder auch der Bewegung beim Abrollen des Fußes, sowie zur Bewegung der Feder während des Gehens oder Laufen, können weiterhin zum Beispiel auch Biegesensoren eingesetzt werden.

Als Sensoren können alle dem Fachmann bekannten und verfügbaren Sensoren genutzt werden. Bei Sensoren, die im Bereich der Laufsohle eingesetzt werden, ist es vorteilhaft, solche einzusetzen, die nur eine geringe Bauhöhe aufweisen, um den Bewegungsablauf nicht zu stören. Weiterhin ist es bevorzugt, im Bereich der Laufsohle biegbare Sensoren zu nutzen, damit die Abrollbewegung des Fußes durch den Sensor nicht eingeschränkt wird.

Die Unterschenkelorthese ist vorzugsweise derart aufgebaut, dass die Feder im Bereich des äußeren Knöchels mit dem Fußteil verbunden ist. Die Feder umläuft dann den Unterschenkel an der Beinhinterseite und ist auf der Beininnenseite mit der den Unterschenkel im Bereich des Schienbeinkopfes umschließenden Manschette verbunden. Durch die den Unterschenkel an der Beinrückseite umschließende Feder wird gegenüber einer geraden Feder, die am Unterschenkel nach oben geführt ist, eine höhere Stabilität des Beins, insbesondere eine seitliche Stabilisierung in der frontalen Beinachse, erreicht. Zudem erlaubt die den Unterschenkel an der Beinrückseite umschließende Feder auch eine unterstützte Drehbewegung des Fußes. Durch die Befestigung der Feder am Fußteil im Bereich des äußeren Knöchels werden weiterhin das obere und das untere Sprunggelenk korrigiert gehalten und sind gleichzeitig dynamisch und beweglich. Durch den spiralförmigen Verlauf ist des weiteren die Beweglichkeit im oberen Sprunggelenk nicht blockiert, sondern alle Abrollbewegungen (Rockerfunktionen) werden unterstützt. Bei einem medialen Kollaps (einer Rotationsfehlstellung) der Beinachse führt die spiralförmige Orthese den Unterschenkel immer wieder dynamisch in die richtige Position. Im Unterschied dazu haben gerade Federn, die meist dorsal am Unterschenkel verlaufen, bei weitem nicht eine so starke Wirkung, da der Kraftvektor der Bodenreaktionskräfte nicht hinter der Ferse verläuft, sondern je nach Gangphase zwischen Fußmitte und Vorfuß. Seitlich verlaufende gerade Federn blockieren in der mittleren Standphase die Bewegung im oberen Sprunggelenk.

Da die Unterschenkelorthese bei verschiedenen Indikationen, die eine Einschränkung des Gehens oder Laufens mit sich bringen, eingesetzt werden kann, und insbesondere gegebenenfalls neben der Stütze des Sprunggelenks auch der Fuß selber eine geschädigt sein kann, kann die Laufsohle Teil einer Fußorthese oder Teil einer knöchelumgreifenden Sprunggelenksorthese sein. Insbesondere bei zusätzlicher Beeinträchtigung des Fußes und der Fußmuskulatur, wie sie beispielsweise bei Muskeldystrophie, neuraler Muskelatrophie oder multipler Sklerose auftreten kann, ist die Laufsohle vorzugsweise Teil einer Fußorthese. Bei Indikationen, die nur das Sprunggelenk betreffen, beispielsweise aufgrund einer Verletzung, ist die Laufsohle vorzugsweise mit einer knöchelumgreifenden Sprunggelenksorthese verbunden. Hierbei darf der Fuß in der Orthese nicht ausweichen oder kompensieren. Je stabiler der Fuß ist, umso weniger muss dieser durch die Sprunggelenkorthese gestützt werden.

Als Material für die Unterschenkelorthese kann jedes, dem Fachmann für den Orthesenbau bekannte Material eingesetzt werden. Besonders bevorzugt wird die Unterschenkelorthese aus einem faserverstärkten Kunststoff hergestellt. Zur Herstellung ist es dabei besonders bevorzugt, die Fasern vorzuimprägnieren, dann zu formen und auszuhärten.

Als Fasern eignen sich zum Beispiel Kohlenstofffasern, Glasfasern, Aramidfasern oder Mineralfasern. Besonders bevorzugt sind Kohlenstofffasern. Als Kunststoff kann jeder beliebige thermoplastische oder duroplastische Kunststoff eingesetzt werden. Bevorzugt werden duroplastische Kunststoffe eingesetzt und besonders bevorzugt sind Epoxidharze.

Durch den Aufbau der Unterschenkelorthese mit Laufsohle, daran angebrachter Feder, die auf der Beinrückseite den Unterschenkel umläuft und die Manschette, mit der die Unterschenkelorthese im Bereich des Schienbeinkopfes fixiert wird, kann ein nahe am gesunden Bewegungsbild liegendes Gangbild erzielt werden. Insbesondere wird durch die Laufsohle und die Befestigung der Unterschenkelorthese im Bereich der Laufsohle die Fußhebung in der Schwungphase unterstützt. Zusätzlich ist auch eine Korrektur von Fußfehlstellungen, insbesondere in Verbindung mit einer Fußorthese, möglich. Eine Korrektur von Fußfehlstellungen kann allerdings auch schon durch die Befestigung der Unterschenkelorthese im Bereich der Laufsohle erreicht werden.

Ein weiterer Vorteil durch die Gestaltung der Unterschenkelorthese ist, dass die Abrollbewegung des Fußes während eines Gangzyklus gesteuert und unterstützt werden kann. Die Konstruktion mit der den Unterschenkel auf der Beinrückseite umlaufenden Feder erlaubt zudem in Verbindung mit dem eingesetzten Material für die Unterschenkelorthese eine verbesserte Kniestreckung und ein leichteres Durchschwingen. Ein ökonomischeres Gehen wird durch die aufgrund der Konstruktion der Unterschenkelorthese erhaltene und im Vergleich zu bekannten Orthesen gesteigerte Energierückgabe erzielt.

Um das Gangbild eines Patienten durch Nutzung der Unterschenkelorthese zu verbessern, umfasst ein Verfahren zur Unterstützung eines Patienten beim Gehen mit einem System zur Unterstützung der Plantarflexion im oberen Sprunggelenk folgende Schritte:
(a) Erfassen von Daten mit den an der Unterschenkelorthese positionierten Sensoren zur Bestimmung eines Bewegungsmusters beim Gehen,
(b) Vergleichen der von den Sensoren erfassten Daten mit gespeicherten Solldaten,
(c) Ausgabe einer Information an den Patienten ob die von den Sensoren erfassten Daten von den Solldaten abweichen oder mit den Solldaten übereinstimmen.

Durch die Erfassung der Daten mit den an der Unterschenkelorthese positionierten Sensoren wird im Augenblick der Bewegung das tatsächliche Bewegungsbild erfasst. Der Vergleich mit den gespeicherten Solldaten erlaubt eine sofortige Rückmeldung an den Patienten, so dass dieser bereits während des gerade ausgeführten Schrittes beim Gehen eine Information erhält, ob der Schritt dem gewünschten Bewegungsmuster entspricht oder von diesem abweicht. Die sofortige Rückmeldung erlaubt eine schnelle Optimierung des Bewegungsbildes. Insbesondere kann bei Rückmeldung einer Abweichung der erfassten Daten von den Solldaten unmittelbar reagiert werden, so dass noch ein Gefühl für die zuvor ausgeführte Bewegung vorhanden ist und auf die tatsächlich zuvor ausgeführte Bewegung reagiert werden kann. Insbesondere kann hierdurch verhindert werden, dass aufgrund zu später oder fehlender Rückmeldung ein falsches Bewegungsmuster eingeübt wird, das nur schwer wieder abtrainiert werden kann.

Die kontinuierliche Rückmeldung an den Patienten erlaubt zudem einen stetigen Lernprozess, der beim Tragen der Unterschenkelorthese einsetzt und dazu führt, dass das Bewegungsmuster selbständig weiter optimiert wird.

Die Information, ob die von den Sensoren erfassten Daten von den Solldaten abweichen oder mit den Solldaten übereinstimmen, kann an ein stationäres Ausgabegerät, beispielsweise einen fest installierten Monitor oder eine Anzeige an einem Laufband, oder an ein mobiles Ausgabegerät übermittelt werden. Bevorzugt erfolgt die Ausgabe an ein mobiles Ausgabegerät. Vorteil eines mobilen Ausgabegerätes ist, dass diese mitgeführt werden kann und auf diese Weise der Trainingseffekt während des Tragens der Unterschenkelorthese ortsunabhängig erzielt werden kann.

Geeignete mobile Ausgabegeräte sind zum Beispiel ein Smartphone, ein Tablet oder ein tragbarer Computer, beispielsweise ein Notebook. In diesem Fall erfolgt die Informationsausgabe an den Patienten und gegebenenfalls auch die Auswertung der Daten in einem mobilen Anwendungsprogramm, einer sogenannten "App".

Die Datenübertragung von den Sensoren an das Ausgabegerät erfolgt vorzugsweise drahtlos, beispielsweise über eine Bluetooth-Schnittstelle oder über ein drahtloses Netzwerk. Hierbei ist es einerseits möglich, dass die einzelnen Sensoren die Daten direkt an eine mit dem Ausgabegerät verbundene oder im Ausgabegerät integrierte Auswerteeinrichtung senden. Die Auswerteeinrichtung kann dabei zum Beispiel auch durch ein auf dem Ausgabegerät laufendes Computerprogramm umgesetzt werden. Bevorzugt ist es, wenn die von den Sensoren erfassten Daten mit einem Mikroprozessor an der Unterschenkelorthese aufgenommen werden und vom Mikroprozessor an das Ausgabegerät geschickt werden. Hierbei ist es einerseits möglich, dass der Mikroprozessor nur die Daten der Sensoren empfängt und in eine zu übertragende Datei schreibt, beispielsweise in Form einer Tabelle, oder dass bereits im Mikroprozessor die Auswertung und der Vergleich mit den Solldaten erfolgt und nur das Ergebnis an die Auswerteeinheit geschickt und von dieser als Information an den Patienten ausgegeben wird. Zur Zwischenspeicherung der Daten bei Einsatz eines Mikroprozessors an der Unterschenkelorthese kann dabei jede beliebige Speichereinrichtung, insbesondere wiederbeschreibbare Speichereinrichtung genutzt werden.

Die von den Sensoren an der Unterschenkelorthese erfassten Daten sind je nach Art der eingesetzten Sensoren zum Beispiel der Druck, der auf die Laufsohle der Unterschenkelorthese beim Aufsetzen des Fußes ausgeübt wird. Weiterhin können zum Beispiel Biegemomente oder Dehnung der Feder mit entsprechenden Biege- oder Dehnungssensoren erfasst werden. Auch ist es möglich, neben dem Druck auch die Biegung der Laufsohle zu erfassen. Zusätzlich kann mit Hilfe von 3D-Bewegungssensoren die Bewegung der Unterschenkelorthese erfasst werden.

Besonders vorteilhaft ist es, die Biegemomente an der Feder und gegebenenfalls der Laufsohle zu erfassen, sobald diese beim Gehen vorgespannt wird. Dies erfolgt, sobald der Patient in der Abstoßphase den Vorfuß richtig belastet. In der Folge kommt es zur Entladung der gespeicherten Energie, wobei die Unterschenkelorthese wie eine Sprungfeder wirkt und so die Bewegung des Beins beim Gehen unterstützt.

Da das optimale Bewegungsbild abhängig von der Indikation, aufgrund derer die Unterschenkelorthese eingesetzt wird, und auch abhängig von der Physiognomie des Patienten ist, werden die Solldaten, mit denen die von den Sensoren erfassten Daten verglichen werden, vorzugsweise für jeden Patienten individuell ermittelt. Die Erfassung erfolgt dabei vorzugsweise mit den Sensoren der Unterschenkelorthese. Damit die erfassten Solldaten dem gewünschten Bewegungsbild entsprechen, werden diese vorzugsweise unter Aufsicht von geschultem Personal, insbesondere unter Aufsicht eines Physiotherapeuten erfasst. Hierzu erfolgt zum Beispiel ein Training mit der Unterschenkelorthese und im Rahmen einer stationären oder ambulanten Therapie, bei der auch das Gehen mit der Unterschenkelorthese geübt wird, werden die Solldaten ermittelt. Die Ermittlung der Solldaten kann alternativ auch bei der Anpassung der Unterschenkelorthese erfolgen.

Da gegebenenfalls die erfassten Solldaten noch nicht dem optimalen Bewegungsbild entsprechen, da eine solche Bewegung vom Patienten noch nicht umgesetzt werden kann, ist es möglich, zunächst Solldaten aufzunehmen, die dem bestmöglichen vom Patienten umsetzbaren Bewegungsbild entsprechen. In diesem Fall können dann, wenn durch das Tragen der Unterschenkelorthese und das Trainieren der Bewegung ein verbessertes Bewegungsbild erzielt worden ist, in einer weiteren Therapiestunde, die ambulant oder stationär durchgeführt werden kann, erneut Solldaten für ein in der Therapie erzieltes weiter verbessertes Bewegungsbild aufgenommen werden, so dass auf diese Weise mit gelegentlichen Therapiestunden und individuellem Training alleine durch das Tragen der Unterschenkelorthese und die Rückmeldung an den Patienten eine iterative Verbesserung des Bewegungsbildes erzielt wird.

Die Information, die an den Patienten übermittelt wird, kann abhängig vom Alter gestaltet werden. So ist es zum Beispiel insbesondere bei minderjährigen Patienten möglich, die Rückmeldung in Form eine Spiels zu gestalten, so dass eine spielerische Verbesserung des Bewegungsbildes erfolgt. Alternativ kann jedoch auch einfach negatives Signal gegeben werden, wenn Abweichungen der erfassten Daten von den Solldaten auftreten und ein positives Signal, wenn die Daten übereinstimmen.

Alternativ zu einer einfachen positiven oder negativen Rückmeldung ist es jedoch auch möglich, für jedes erfasste Signal die tatsächliche Abweichung anzugeben. Auf diese Weise ließe sich zum Beispiel bei einem konzentrierten Training gerade auf Verbesserung der spezifischen Bewegungen abstellen, bei denen die Abweichung besonders groß ist. Dies ist jedoch nur im Rahmen eines spezifischen Gehtrainings möglich, da die Erfassung der einzelnen Daten durch den Patienten zu umständlich ist, um während des normalen Gehens umgesetzt zu werden. Bevorzugt ist daher eine einfache Rückmeldung durch ein positives oder negatives Signal gegebenenfalls mit einem kurzen Hinweis, was genau am Gang geändert werden sollte, um eine Verbesserung zu erhalten. Wenn nur ein positives oder negatives Signal gegeben wird, liegt es am Patienten durch Änderung der Bewegung eine Verbesserung des Gangbildes zu erhalten.

Da das Gangbild üblicherweise zunächst mit einem Therapeuten geübt wird, sollte eine solche positive oder negative Rückmeldung jedoch ausreichen, da aus den Therapiestunden in der Regel ein ausreichendes Gefühl für die Bewegung mitgenommen wird, so dass die Erinnerung an die Therapiestunde und die dort erhaltenen Informationen ausreichen sollten, bei einer negativen Rückmeldung eine Verbesserung des Bewegungsbildes umzusetzen.

Bei der Prüfung der Übereinstimmung der Solldaten mit den von den Sensoren erfassten Daten ist immer auch eine Toleranz zu berücksichtigen. Diese wird so gewählt, dass ein zwar noch nicht optimales Bewegungsbild, aber eines, welches weitgehend dem optimalen Bewegungsbild entspricht, bereits mit einer positiven Rückmeldung verbunden ist. Wie groß die einzelnen Toleranzen sein dürfen hängt dabei auch von den eingesetzten Sensoren ab. Vorteilhaft ist, wenn durch die Solldaten jeweils ein Bereich definiert wird, innerhalb dessen die Messwerte der jeweiligen Sensoren liegen, wenn das gewünschte Bewegungsbild erhalten wird. So wird durch die Solldaten für jeden Sensor jeweils ein Bereich zwischen einem Minimalwert und einem Maximalwert vorgegeben und solange sich der von dem Sensor erfasste Wert innerhalb des Bereichs zwischen Minimalwert und Maximalwert befindet, ist eine Übereinstimmung des vom Sensor erfassten Werts mit den Solldaten gegeben und es wird eine positive Rückmeldung gegeben.

Ein Ausführungsbeispiel der Erfindung ist in der Figur dargestellt und wird in der nachfolgenden Beschreibung näher erläutert.

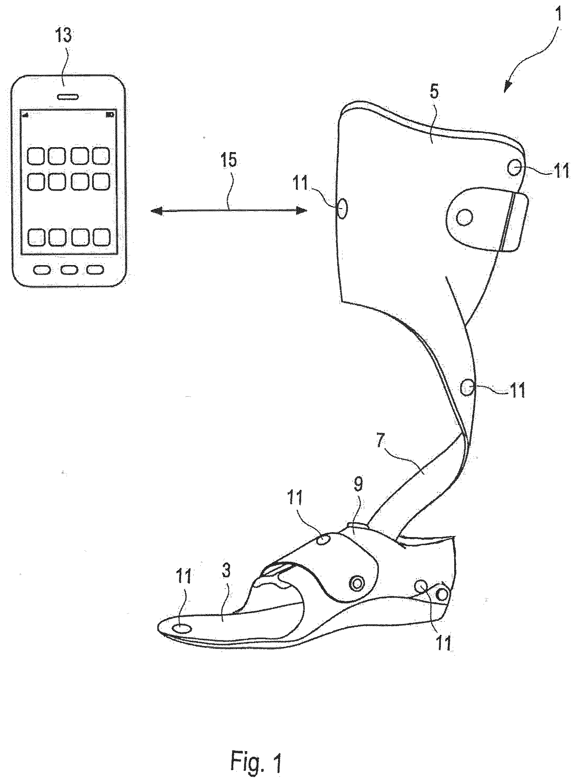

Figur 1 zeigt ein erfindungsgemäßes System mit Unterschenkelorthese und Auswerteeinheit.

Eine Unterschenkelorthese 1 umfasst eine Laufsohle 3, eine Manschette 5, mit der die Unterschenkelorthese im Bereich des Schienbeinkopfs am Bein eines Patienten befestigt werden kann sowie eine Feder 7, die sich von der Laufsohle 3 zur Manschette 5 hin erstreckt und den Unterschenkel auf der Beinrückseite spiralförmig umschließt. Die Feder 7 ist dabei vorzugsweise derart gestaltet, dass diese vom äußeren Knöchel zur Beininnenseite auf Höhe des Schienbeinkopfes verläuft. Zur Befestigung der Unterschenkelorthese 1 am Bein eines Patienten ist zusätzlich zur Manschette 5 eine Befestigungseinrichtung 9 an der Laufsohle 3 vorgesehen. Um einen passgenauen Sitz der Unterschenkelorthese 1 zu erhalten, sind vorzugsweise sowohl die Manschette 5 zur Befestigung der Unterschenkelorthese 1 im Bereich des Schienbeinkopfes als auch die Befestigungseinrichtung 9 an der Laufsohle 3 mit einstellbaren Verschlüssen versehen. Als einstellbare Verschlüsse eignen sich dabei insbesondere Klettverschlüsse.

Erfindungsgemäß ist die Unterschenkelorthese mit mindestens einem Sensor 11, vorzugsweise jedoch mit mehreren Sensoren 11 ausgestattet, wobei mit Hilfe der Sensoren 11 Daten erfasst werden, mit denen ein Bewegungsmuster erfasst werden kann. Als Sensoren können zum Beispiel Drucksensoren, Biegesensoren, Dehnungssensoren, Bewegungssensoren oder auch Elektroden zur Erfassung der Muskelaktivität eingesetzt werden.

Bevorzugt ist es, im Bereich der Laufsohle 3 Drucksensoren und im Bereich der Feder 7 Biege- und/oder Dehnungssensoren einzusetzen. Zusätzlich können an einer beliebigen Stelle der Unterschenkelorthese 1, beispielsweise an der Laufsohle 3, an der Manschette 5 oder an der Feder 7 Bewegungssensoren angebracht sein. Wenn zusätzlich Elektroden zur Erfassung der Muskelaktivität vorgesehen sind, so werden diese vorzugsweise an der Manschette 5 angebracht.

Die von den Sensoren 11 erfassten Daten werden einer Auswerteeinheit zugeführt, in der die Daten mit Solldaten verglichen werden. Die Auswerteeinheit kann dabei entweder direkt an der Unterschenkelorthese angebracht sein, zum Beispiel in Form eines Mikroprozessors, insbesondere im Bereich der Manschette 5. Alternativ kann die Auswerteeinheit jedoch auch als von der Unterschenkelorthese 1 getrennte Einheit ausgebildet sein.

Durch den Vergleich mit den Solldaten wird ein Hinweis auf das aktuelle Bewegungsmuster erhalten. Wenn die von den Sensoren 11 erfassten Daten mit den Solldaten übereinstimmen, entspricht die ausgeführte Bewegung dem gewünschten Bewegungsmuster. Wenn die von den Sensoren 11 erfassten Daten nicht mit den Solldaten übereinstimmen, muss eine Korrektur der Bewegung erfolgen. Hierzu erhält der Patient eine Information auf einem Ausgabegerät 13.

Das Ausgabegerät 13 kann ein mobiles Ausgabegerät sein, wie es hier dargestellt ist, oder ein stationäres Ausgabegerät, beispielsweise ein Monitor oder eine Anzeigeeinheit an einem Laufband. Als mobile Ausgabegeräte 13 eignen sich zum Beispiel Smartphones oder Tablets.

Wenn die Auswerteeinheit nicht an der Unterschenkelorthese 1 angebracht ist sondern ein separates Bauteil ist, kann die Auswerteeinheit zum Beispiel auch eine Einheit mit dem Ausgabegerät 13 bilden.

Bei einer als separatem Bauteil ausgeführten Auswerteeinheit werden die von den Sensoren 11 erfassten Daten an die Auswerteeinheit gesendet, vorzugsweise über eine drahtlose Verbindung und dann in der Auswerteeinheit ausgewertet, das heißt mit den hinterlegten Solldaten verglichen. Das Ergebnis dieses Vergleichs wird dann in der Anzeigeeinheit 13 dargestellt.

Bei einer an der Unterschenkelorthese 1 angebrachten Auswerteeinheit können die von den Sensoren 11 erfassten Daten ebenfalls drahtlos übertragen werden. Alternativ ist es in diesem Fall jedoch möglich, die Daten von den Sensoren auch über Kabel, die in der Unterschenkelorthese 1 von den Sensoren 11 zur Auswerteeinheit verlaufen, zu übertragen. In der Auswerteeinheit erfolgt dann der Vergleich mit den Solldaten und das Ergebnis wird an das Ausgabegerät 13 gesendet.

In einer weiteren Ausführungsform ist es auch möglich, die von den Sensoren 11 erfassten Daten in einer Datenerfassungseinheit in der Unterschenkelorthese 1 zu sammeln und von dieser gesammelt an die Auswerteeinheit 1 zu übertragen. Dies hat den Vorteil, dass nicht jeder Sensor getrennt Daten sendet und die Auswerteeinheit drahtlos von mehreren Sendern Daten empfängt.

Unabhängig von der Art der Datenübertragung, das heißt Übertragung der von den Sensoren erfassten Daten an eine externe Auswertevorrichtung, wobei die Daten entweder direkt von den Sensoren 11 oder alternativ von einer Datenerfassungseinheit gesendet werden, oder Übertragung des Ergebnisses von einer an der Unterschenkelorthese 1 angebrachten Auswerteeinheit an die Ausgabeeinheit 13, ist die Datenübermittlung hier schematisch mit einem Pfeil 15 dargestellt.

Um sicherzustellen, dass die Ausgabeeinheit 13 auch die Daten der Sensoren 11 der Unterschenkelorthese 1 oder der an der Unterschenkelorthese 1 angebrachten Auswerteeinheit empfängt, ist es vorteilhaft, wenn entweder die Sensoren 11 oder die Auswerteeinheit, die an der Unterschenkelorthese 1 angebracht ist, ein Signal von der Ausgabeeinheit 13 empfangen können, mit dem mitgeteilt wird, dass das Gerät vorhanden und funktionsbereit ist.

## Patentansprüche

1. System zur Unterstützung der Plantarflexion im oberen Sprunggelenk, umfassend eine Unterschenkelorthese (1) mit einem Fußteil mit einer Laufsohle (3) sowie eine Manschette (5) zur Befestigung der Unterschenkelorthese (1) im Bereich des Schienbeinkopfs, wobei das Fußteil und die Manschette (5) mit einer den Unterschenkel an der Beinhinterseite umschließenden Feder (7) verbunden sind, wobei an der Unterschenkelorthese (1) Sensoren (11) zur Erfassung eines Bewegungsmusters positioniert sind und das System eine Auswerteeinheit umfasst, die derart eingerichtet ist, dass von den Sensoren (11) erfasste Daten zur Erfassung des Bewegungsmusters mit Solldaten verglichen werden und bei Abweichung eine Information an den Träger der Unterschenkelorthese gegeben wird, **dadurch gekennzeichnet, dass** die Feder (7) im Bereich des äußeren Knöchels mit dem Fußteil verbunden ist, den Unterschenkel an der Beinhinterseite umläuft und auf der Beininnenseite mit der den Unterschenkel im Bereich des Schienbeinkopfs umschließenden Manschette verbunden ist.

2. System gemäß Anspruch 1, **dadurch gekennzeichnet, dass** die Sensoren (11) zur Erfassung des Bewegungsmusters ausgewählt sind aus Drucksensoren, Biegesensoren, Dehnungssensoren, Bewegungssensoren und Elektroden zur Messung der elektrischen Muskelaktivität.

3. System gemäß Anspruch 1 oder 2, **dadurch gekennzeichnet, dass** an der Laufsohle (3) Drucksensoren und an der Feder Dehnungssensoren und Bewegungssensoren angebracht sind.

4. System gemäß einem der Ansprüche 1 bis 3, **dadurch gekennzeichnet, dass** Elektroden zur Messung der elektrischen Muskelaktivität an der Manschette (5) angebracht sind.

5. System gemäß einem der Ansprüche 1 bis 4, **dadurch gekennzeichnet, dass** die Laufsohle (3) Teil einer Fußorthese oder Teil einer knöchelumgreifenden Sprunggelenksorthese ist.

6. System gemäß einem der Ansprüche 1 bis 5, **dadurch gekennzeichnet, dass** die Information an ein mobiles Ausgabegerät (13) übermittelt wird.

7. System gemäß Anspruch 6, **dadurch gekennzeichnet, dass** das mobile Ausgabegerät (13) ein Smartphone oder ein Tablet ist.

8. System gemäß einem der Ansprüche 1 bis 7, **dadurch gekennzeichnet, dass** die Solldaten individuell mit den Sensoren (11) der Unterschenkelorthese (1) erfasst werden.

9. System gemäß einem der Ansprüche 1 bis 8, **dadurch gekennzeichnet, dass** die Information bei Übereinstimmung der von den Sensoren (11) erfassten Daten mit den Solldaten eine positive Rückmeldung ist.

10. System gemäß einem der Ansprüche 1 bis 9, **dadurch gekennzeichnet, dass** die Information bei fehlender Übereinstimmung der von den Sensoren (11) erfassten Daten mit den Solldaten eine negative Rückmeldung ist.

11. System gemäß einem der Ansprüche 1 bis 10, **dadurch gekennzeichnet, dass** die Solldaten jeweils einen Bereich zwischen einem Minimalwert und einem Maximalwert bezeichnen und eine Übereinstimmung gegeben ist, wenn der vom jeweiligen Sensor erfasste Wert zwischen dem Minimalwert und dem Maximalwert liegt.

## Claims

1. System for assisting plantar flexion in the upper ankle joint, comprising a below-knee orthosis (1) having a foot part with an outsole (3), and a cuff (5) for securing the below-knee orthosis (1) in the region of the head of the tibia, wherein the foot part and the cuff (5) are connected to a spring (7) enclosing the lower leg on the posterior aspect thereof, wherein sensors (11) for detecting a movement pattern are positioned on the below-knee orthosis (1), and the system comprises an evaluation unit which is configured in such a way that data detected by the sensors (11) for detection of the movement pattern are compared with target data and, in the event of deviation, information is output to the wearer of the below-knee orthosis, **characterized in that** the spring (7) is connected to the foot part in the region of the lateral malleolus, runs round the posterior aspect of the lower leg and, on the inner aspect, is connected to the cuff enclosing the lower leg in the region of the head of the tibia.

2. System according to Claim 1, **characterized in that** the sensors (11) for detecting the movement pattern are chosen from pressure sensors, bend sensors, elongation sensors, motion sensors, and electrodes for measurement of electrical muscle activity.

3. System according to Claim 1 or 2, **characterized in that** pressure sensors are mounted on the outsole (3), and elongation sensors and motion sensors are mounted on the spring.

4. System according to one of Claims 1 to 3, **characterized in that** electrodes for measurement of electrical muscle activity are mounted on the cuff (5).

5. System according to one of Claims 1 to 4, **characterized in that** the outsole (3) is part of a foot orthosis or part of an ankle joint orthosis engaging around the malleolus.

6. System according to one of Claims 1 to 5, **characterized in that** the information is transmitted to a mobile output device (13).

7. System according to Claim 6, **characterized in that** the mobile output device (13) is a smart phone or a tablet.

8. System according to one of Claims 1 to 7, **characterized in that** the target data are detected individually with the sensors (11) of the below-knee orthosis (1).

9. System according to one of Claims 1 to 8, **characterized in that**, if the data detected by the sensors (11) corresponds to the target data, the information is a positive feedback.

10. System according to one of Claims 1 to 9, **characterized in that**, if the data detected by the sensors (11) fails to correspond to the target data, the information is a negative feedback.

11. System according to one of Claims 1 to 10, **characterized in that** the target data each designate a range between a minimum value and a maximum value, and the values correspond when the value detected by the respective sensor lies between the minimum value and the maximum value.

## Revendications

1. Système de support de la flexion plantaire dans le jarret supérieur, comprenant une orthèse de jambe (1) dotée d'un élément de pied doté d'une semelle extérieure (3) ainsi que d'un manchon (5) pour fixer l'orthèse de jambe (1) dans la zone du plateau tibial, dans lequel l'élément de pied et le manchon (5) sont reliés à un ressort (7) entourant la jambe au plateau tibial, dans lequel des capteurs (11) sont positionnés sur l'orthèse de jambe (1) afin de saisir un ensemble de mouvements et le système comprend une unité de traitement, laquelle est disposée de sorte que des données saisies par les capteurs (11) afin de saisir l'ensemble de mouvements sont comparées à des données de consigne et une information est donnée au porteur de l'orthèse de jambe en cas de déviation, **caractérisé en ce que** le ressort (7) est relié à l'élément de pied dans la zone extérieure de la cheville, entoure la jambe au jarret et est relié sur la face intérieure de la jambe au manchon entourant la jambe dans la zone du plateau tibial.

2. Système selon la revendication 1, **caractérisé en ce que** les capteurs (11) destinés à la saisie de l'ensemble de mouvements sont sélectionnés parmi les capteurs de pression, les capteurs de flexion, les capteurs de contrainte, les capteurs de mouvement et les électrodes de mesure de l'activité musculaire électrique.

3. Système selon la revendication 1 ou 2, **caractérisé en ce que** des capteurs de pression sont arrangés sur la semelle extérieure (3) et des capteurs de contrainte et des capteurs de mouvement sont arrangés sur le ressort.

4. Système selon l'une des revendications 1 à 3, **caractérisé en ce que** des électrodes de mesure de l'activité musculaire électrique sont arrangées sur le manchon (5).

5. Système selon l'une des revendications 1 à 4, **caractérisé en ce que** la semelle extérieure (3) est une partie d'une orthèse de pied ou une partie d'une orthèse de jarret enveloppant la cheville.

6. Système selon l'une des revendications 1 à 5, **caractérisé en ce que** l'information est transmise à un périphérique de sortie mobile (13).

7. Système selon la revendication 6, **caractérisé en ce que** le périphérique de sortie mobile (13) est un smartphone ou une tablette.

8. Système selon l'une des revendications 1 à 7, **caractérisé en ce que** les données de consignes sont saisies individuellement avec les capteurs (11) de l'orthèse de jambe (1).

9. Système selon l'une des revendications 1 à 8, **caractérisé en ce que** l'information en cas de concordance des données saisies par les capteurs (11) avec les données de consigne est une rétroaction positive.

10. Système selon l'une des revendications 1 à 9, **caractérisé en ce que** l'information en cas de non-concordance des données saisies par les capteurs (11) avec les données de consigne est une rétroaction négative.

11. Système selon l'une des revendications 1 à 10, **caractérisé en ce que** les données de consigne désignent respectivement un intervalle entre une valeur minimale et une valeur maximale et il existe une concordance lorsque la valeur saisie par le capteur respectif est située entre la valeur minimale et la valeur maximale.
